# EUROPEAN PATENT APPLICATION

(11) **EP 4 067 877 A1**
(43) Date of publication of application: **05.10.2022**
(21) Application number: 20894901.6
(22) Date of filing: 26.11.2020
(51) Int. Cl.: G01N 21/49

(54) **OIL MIST DETECTION DEVICE**

(30) Priority: 29.11.2019 JP 2019217277
(71) Applicant: DAIHATSU DIESEL MFG. CO., LTD., Osaka 531-0076 (JP); HORIBA, Ltd., Kyoto-shi, Kyoto 601-8510 (JP)
(72) Inventor: MOCHIZUKI, Masaru, Osaka-shi, Osaka 531-0076 (JP); ABE, Noboru, Osaka-shi, Osaka 531-0076 (JP); INOUE, Kentaro, Kyoto-shi, Kyoto 601-8510 (JP); MATSUNAGA, Shimpachi, Kyoto-shi, Kyoto 601-8510 (JP); MATANO, Toshihiko, Kyoto-shi, Kyoto 601-8510 (JP)
(74) Representative: Müller Hoffmann & Partner
(86) International application number: PCT/JP2020/044003
(87) International publication number: WO 2021/107004

(57) **Abstract**

To provide an explosion-proof oil mist detection device without incurring high costs, an oil mist detection device 100 for detecting oil mist in a casing 11 comprises a sensor unit 12 that is housed in the casing 11 and arranged in an explosion-proof area (X) that requires explosion-proof measures, a wiring member (P) that is connected to the sensor unit 12, a barrier circuit 30 that is connected to the sensor unit 12 through the wiring member (P), and a bulkhead member 40 through which the wiring member (P) penetrates and that is housed in the casing 11 and that seals the explosion-proof area. The oil mist detection device 100 is so configured that the barrier circuit 30 is arranged in a non-explosion-proof area (Y) that is isolated from the explosion-proof area (X) by the bulkhead member 40.

## Description

### Field of the art

This invention relates to an oil mist detection device that detects oil mist.

### Background art

As shown in Patent Document 1, there is an oil mist detection device of this type that is mounted on, for example, a crankcase of an internal combustion engine for ship and that detects oil mist generated in the crankcase.

In recent years, environmental regulations have led to the use of a natural gas as fuel for internal combustion engines in place of a conventional heavy oil. In such cases, since the oil mist detection device is exposed to an explosive (flammable) natural gas, it is required to take explosion-proof measures. As concrete measures, for example, it is required to adopt an intrinsically safe explosion-proof structure, in which a barrier circuit is required to be provided to limit the energy supplied to a sensor unit of the oil mist detection device.

However, in case of using a device with a barrier circuit that is separated from the oil mist detection device, a manufacturing cost of the separated device would be high. In addition, even if a conventional oil mist detection device comprises a barrier circuit, since the barrier circuit is in danger of being exposed to the natural gas, resulting in failure of meeting the requirements.

### Prior art documents

### Patent documents

Patent document 1: Japanese unexamined patent application publication No. 2008-157648

### Disclosure of the invention

### Problems to be solved by the invention

A main object of this invention is to provide an oil mist detection device having unprecedentedly explosion-proof specification without incurring a high cost.

### Means to solve the problems

The oil mist detection device in accordance with the present claimed invention is an oil mist detection device that detects oil mist in a casing and that comprises a sensor unit that is housed in the casing and arranged in an explosion-proof area that requires explosion-proof measures, a wiring member that is connected to the sensor unit, a barrier circuit that is connected to the sensor unit through the wiring member, and a bulkhead member through which the wiring member penetrates and that is housed in the casing and that seals the explosion-proof area, and is characterized by that the barrier circuit is arranged in a non-explosion-proof area that is isolated from the explosion-proof area by the bulkhead member.

In accordance with the oil mist detection device having the above-mentioned configuration, since the barrier circuit is arranged in the non-explosion-proof area, it is possible to implement the barrier circuit and the sensor unit that is required to be explosion-proof in a single device.

With this arrangement, there is no need of manufacturing a device comprising a barrier circuit separated from the oil mist detection device so that it is possible to provide the oil mist detection device of the explosion-proof specification without incurring higher costs.

For example, in case that a twisted wire consisting of multiple conductors is used to connect the sensor unit to the barrier circuit, a gas in the crankcase might slip through the gaps between a plurality of the conductors that constitute the twisted wire and might flow from the explosion-proof area into the non-explosion-proof area.

In order to prevent the gas from slipping through, it is preferable that a plurality of the wiring members connect the sensor unit and the barrier circuit in a state wherein each of the wiring members is non-contact.

In order to prevent the gas from slipping though more securely, it is preferable that the wiring member is a single wire.

Even in case that the wiring member is covered with an insulating member, the gas in the crank case might slip through between the gap between the insulating member and the wiring member, Then, it is preferable that the wiring member connects the sensor unit and the barrier circuit without being covered by an insulating member.

If there is a gap between the wiring member and the bulkhead member, the gas in the crank will flow from the explosion-proof area to the non-explosion-proof area through the gap. Then, it is preferable that the wiring member airtightly penetrates the bulkhead member.

It is preferable that the bulkhead member is an elastic body made of resin.

In accordance with this arrangement, it is possible to allow the bulkhead member to adhere closely to the casing so that the explosion-proof area can be sealed securely.

As an embodiment of the barrier circuit that meets requirements for explosion-proof measures represented is the barrier circuit that limits the energy supplied to the sensor unit.

### Effect of the Invention

In accordance with the present claimed invention having the above-mentioned configuration, it is possible to provide an unprecedented explosion-proof oil mist detection device without incurring high costs.

### Brief description of the drawings

[Fig. 1] A schematic diagram showing an overall configuration of an oil mist detection device of this embodiment.
[Fig. 2] A schematic diagram showing an internal configuration of the oil mist detection device of this embodiment.
[Fig. 3] A circuit diagram showing a configuration of a barrier circuit of this embodiment.
[Fig. 4] A schematic diagram showing a shape of a bulkhead member of other embodiments.
[Fig. 5] A schematic diagram showing an internal configuration of the oil mist detection device of the other embodiment.

### Explanation of the code

- 100: oil mist detection device
- C: crankcase
- 10: detection section
- 10a: inlet port
- 10b: outlet port
- 10c: oil mist inlet chamber
- 10d: sensor housing chamber
- 11: casing
- 12: sensor unit
- 13: light emitting section
- 14: light receiving section
- 15: substrate
- W: translucent window
- 20: control section
- 21: computer circuit
- 22: second casing
- P: wiring member
- 30: barrier circuit
- 40: bulkhead member
- X: explosion-proof area
- Y: non-explosion-proof area

### Best modes of embodying the invention

One embodiment of an oil mist detection device in accordance with the present claimed invention is explained below with reference to drawings.

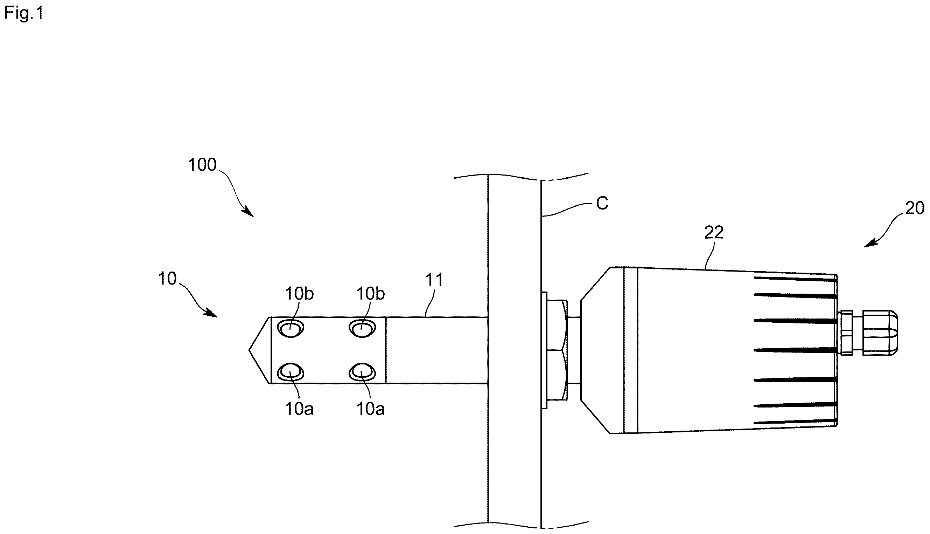

As shown in Fig. 1, the oil mist detection device 100 of this embodiment is a light scattering type that is mounted on a crankcase (C) of an internal combustion engine, for example, for ship and is used to detect oil mist generated in the crankcase (C) due to overheating of a bearing. The application of the oil mist detection device 100 is not limited to this, and it may also be used on internal combustion engines other than ships.

Concretely, the oil mist detection device 100 comprises a detection section 10 that is mounted on a mounting bore arranged on a wall of the crank case (C) from the outside in a state wherein a distal end side of the detection section 10 protrudes to an inside of the crankcase (C) and a control section 20 that is connected to a proximal end of the detection section 10.

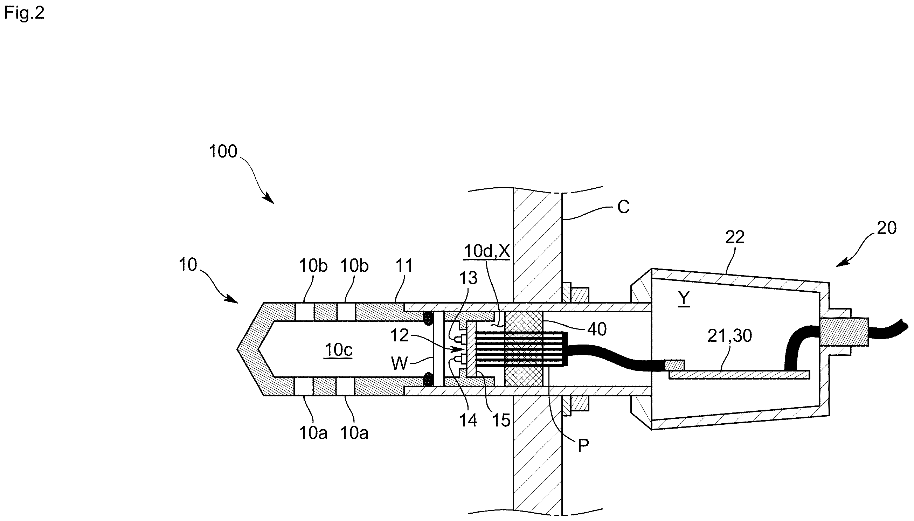

As shown in Fig. 1 and Fig. 2, the detection section 10 has a casing 11 and a sensor unit 12 that is housed in the casing 11.

The casing 11 is in a generally cylindrical shape with a sealed distal end surface and has an oil mist inlet port 10a that leads the oil mist to the inside of the casing 11 and an oil mist outlet port 10b that is formed to discharge the oil mist to the outside thereof. Screw threads (not shown in drawings) that are screwed into the mounting bore of the crankcase (C) are formed on an outer peripheral surface of the casing 11.

As shown in Fig. 2, an oil mist inlet chamber 10c is formed on the distal end side of the casing 11 to introduce and diffuse the oil mist into the casing 1, and a sensor housing chamber 10d is formed on the proximal end side of the casing 11 to accommodate the sensor unit 12. A translucent window (W) such as a lens is interposed between the oil mist inlet chamber 10c and the sensor housing chamber 10d so that the oil mist is prevented from flowing from the oil mist inlet chamber 10c into the sensor housing chamber 10d.

The sensor unit 12 detects the oil mist introduced into the oil mist inlet chamber 10c and comprises a light emitting section 13 and a light receiving section 14.

The light emitting section 13 is, for example, LEDs with its respective light emitting surface arranged to face the translucent window (W). In this embodiment used is the light emitting section 13 that emits wavelength band light that conforms to a particle size of the oil mist. It is a matter of course that other light emitting section 13 such as LDs (laser diodes) may also be used.

The light receiving section 14 is, for example, PDs (photo diodes) with its respective light receiving surface arranged to face the translucent window (W) and outputs an electrical signal corresponding to an intensity of the light received on its light receiving surface. It is a matter of course that other light receiving section 14 such as, for example, CCDs may also be used.

The irradiation light emitted from the light emitting section 13 is irradiated to the oil mist introduced into the oil mist inlet chamber 10c through the translucent window (W), and the scattered light produced by this is received by the light receiving section 14 after passing through the translucent window (W).

The light emitting section 13 and the light receiving section 14 are arranged in parallel so that the optical axes of the emitted light and the optical axis of the received light are parallel to each other, and the light emitting section 13 and the light receiving section 14 are mounted, for example, on an identical same substrate 15. However, the optical axes of the light emitting section 13 and light receiving section 14 are not necessarily parallel, and the light emitting section 13 and light receiving section 14 may be mounted on separate substrates.

The substrate 15, together with the light emitting section 13 and the light receiving section 14, constitutes the sensor unit 12 and is housed in the sensor housing chamber 10d. One or more wiring members (P) are connected to this substrate 15, and the sensor unit 12 and the control section 20 are connected by the wiring members (P).

One or more wiring members (P) are provided. In this embodiment, some wiring member (P) is to supply energy (current and/or voltage) from the control section 20 to the light emitting section 13 and some wiring member (P) is to transmit a detection signal which is an electrical signal corresponding to the intensity of the scattered light received by the light receiving section 14 to the control section 20.

The control section 20 is structurally a so-called computer circuit 21 having a CPU, an internal memory, an AD converter or the like. The control section 20 performs information processing by operating according to programs stored in a predetermined area of the internal memory. In this embodiment, the control section 20 detects whether there is the oil mist or not based on the detection signal transmitted from the light receiving section 14 or measures the concentration of the oil mist based on the detection signal.

The control section 20 further comprises a second casing 22 that houses the above-mentioned computer circuit 21. An internal space of the second casing 22 forms the sensor housing chamber 10d by communicating with the internal space of the casing 11 that constitutes the above-mentioned detection section 10. In this embodiment, the second casing 22 has an approximate cylindrical shape, however, it may also have, for example, a rectangular shape.

In case that an explosive (flammable) gas such as a natural gas is used as the fuel for ships, since the explosive gas is supplied to the inside of the crankcase (C), at least a part arranged in the crankcase (C), into which the explosive gas can flow, of the oil mist detection device 100, more concretely a part in the crankcase (C) into which the explosive gas can flow is an explosion-proof area (X) where explosion-proof measures are required.

If explained in more detail, in case that the explosive gas is supplied to the crankcase (C), since the explosive gas is introduced into the above-mentioned oil mist inlet chamber 10c, the oil mist inlet chamber 10c is the explosion-proof area (X) that requires explosion-proof measures. In this embodiment, since there is no component constituting the oil mist inlet chamber 10c that requires explosion-proof measures, there is no need of taking concrete explosion-proof measures for the oil mist inlet chamber 10c.

On the other hand, although the sensor housing chamber 10d and the oil mist inlet chamber 10c are partitioned by the translucent window (W), there is a small gap through which the gas can pass and that is interposed between the sensor housing chamber 10d and the oil mist inlet chamber 10c. With this arrangement, since the explosive gas flows into the sensor housing chamber 10d, the sensor housing chamber 10d also is the explosion-proof area (X) that requires explosion-proof measures. In addition, since the sensor unit 12 is arranged in this sensor housing chamber 10d, and the light emitting section 13, the light receiving section 14, and the substrate 15 that constitute the sensor unit 12 can be an ignition source, the concrete intrinsically safe explosion-proof measures are required for the sensor housing chamber 10d.

As shown in Fig. 2, the oil mist detection device 100 of this embodiment further comprises a barrier circuit 30 that is connected to the sensor unit 12 through the wiring member (P) and that limits the energy supplied to the sensor unit 12.

The barrier circuit 30 of this embodiment is arranged on the same substrate as the computer circuit 21 that constitutes the above-mentioned control section 20 and is housed in the second casing 22. The barrier circuit 30 may be arranged on a substrate separated from the computer circuit 21.

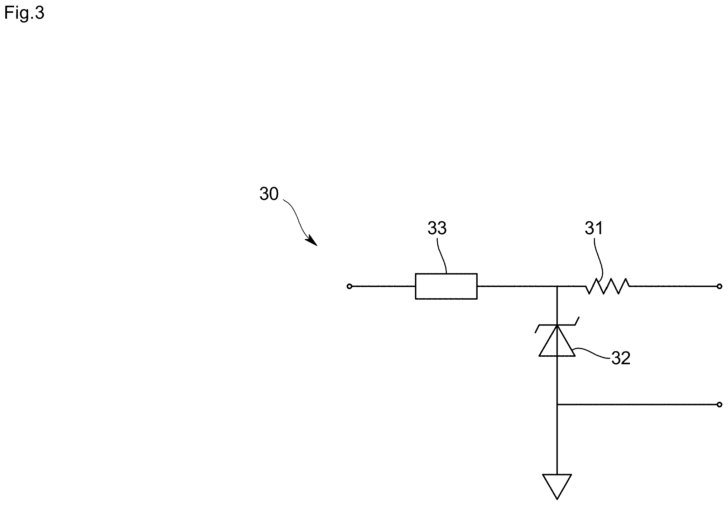

The barrier circuit 30 is to secure the intrinsically safe explosion-proof structure of the oil mist detection device 100 and comprises a function to limit the energy supplied to the sensor unit 12 so that various electronic components that constituts the sensor unit 12 cannot be an ignition source. Concretely, as shown in Fig. 3, the barrier circuit 30 has at least a current limiting element 31 that limits the current flowing in the sensor unit 12 to a predetermined current value or less and a voltage limiting element 32 that limits the voltage applied to the sensor unit 12 to a predetermined voltage value or less. In this embodiment, the barrier circuit 30 further has a protection element 33 that protects the voltage limiting element 32.

Then, the oil mist detection device 100 of this embodiment further comprises a bulkhead member 40 that seals the explosion-proof area (X) by being housed in the above-mentioned casing 11 as shown in Fig. 2 and the barrier circuit 30 is arranged in the non-explosion-proof area (Y) that is isolated from the explosion-proof area (X) by the bulkhead member 40.

If explained in more detail, the bulkhead member 40 airtightly seals the oil mist inlet chamber 10c and the sensor housing chamber 10d as being the explosion-proof area (X) while the above-mentioned wiring member (P) penetrates the bulkhead member 40. Concretely, the bulkhead member 40 is a roughly cylindrical shape arranged in the casing 11 so that a whole circumference of the outer peripheral surface of the bulkhead member 40 airtightly contacts an inner peripheral surface of the casing 11.

In addition, the bulkhead member 40 of this embodiment is an elastic material made of resin such as a liquid gasket that becomes silicone rubber when cured and an outer diameter of the bulkhead member 40 tightly attaches to the inner wall of the casing 11. A material of the bulkhead member 40 is not limited to this, and may be changed as needed, for example, to glass or ceramics, as far as the material does not pass the gas.

An inside of the oil mist detection device 100 (an inside of the casing 11 and an inside of the second casing 22) is partitioned by the bulkhead member 40 into the explosion-proof area (X) locating in a distal end side from the bulkhead member 40 and the non-explosion-proof area (Y) locating in a proximal end side from the bulkhead member 40. In this embodiment, the explosion-proof area (X) is an area arranged inside the crankcase (C), and the non-explosion-proof area (Y) is an area arranged outside the crankcase (C). With this arrangement, no gas circulates between the explosion-proof area (X) and the non-explosion-proof area (Y). The above-mentioned barrier circuit 30 is arranged in the non-explosion-proof area (Y).

The oil mist detection device 100 of this embodiment is so configured to prevent the gas from flowing from the explosion-proof area (X) to the non-explosion-proof area (Y) caused by the wiring member (P).

Concretely, if the wiring member (P) is, for example, a twisted wire consisting of a plurality of conductors (wires) twisted together, the gas in the crankcase (C) slips through a gap between a plurality of the conductors (wires) that constitute the twisted wire and flows from the explosion-proof area (X) to the non-explosion-proof area (Y).

Then in this embodiment, a plurality of the wiring members (P) connect the sensor unit 12 and the barrier circuit 30 without the wiring members (P) being in touch each other, namely, in state wherein each of the wiring members is non-contact.

More concretely, the wiring members (P) are linear or pin-shaped metal referred to as a single wire or a single needle and used in a state that the surface of the wiring member (P) is exposed without being covered by a coating material such as an insulating member.

All of the outer peripheral surface of the wiring members (P) airtightly make contact with the inner peripheral surface of a through bore formed in the bulkhead member 40 so that the wiring members (P) penetrate the bulkhead member 40 without any gap through which the gas can pass.

In accordance with the oil mist detection device 100 having the above-mentioned configuration, since the barrier circuit 30 is arranged in the non-explosion-proof area (Y), it is possible to implement the barrier circuit 30 and the sensor unit 12, which require explosion-proof measures, in a single device.

With this configuration, there is no need of manufacturing a device comprising a barrier circuit 30 separated from the oil mist detection device 100 so that it is possible to provide the oil mist detection device 100 of the explosion-proof specification without incurring higher costs.

In addition, since a single wire or a single needle that does not contact each other is used as a plurality of the wiring members (P) without using twisted wires, it is possible to prevent the gas from slipping through that occurs in case that twisted wires are used. In addition, since the wiring members (P) airtightly penetrate the bulkhead member 40 without being covered by a covering member, it is possible to securely prevent the gas from flowing from the explosion-proof area (X) to the non-explosion-proof area (Y).

Furthermore, since the bulkhead member 40 is the elastic body made of resin and has good adhesion, it is possible to securely seal the explosion-proof area (X).

The invention is not limited to the above-mentioned embodiment.

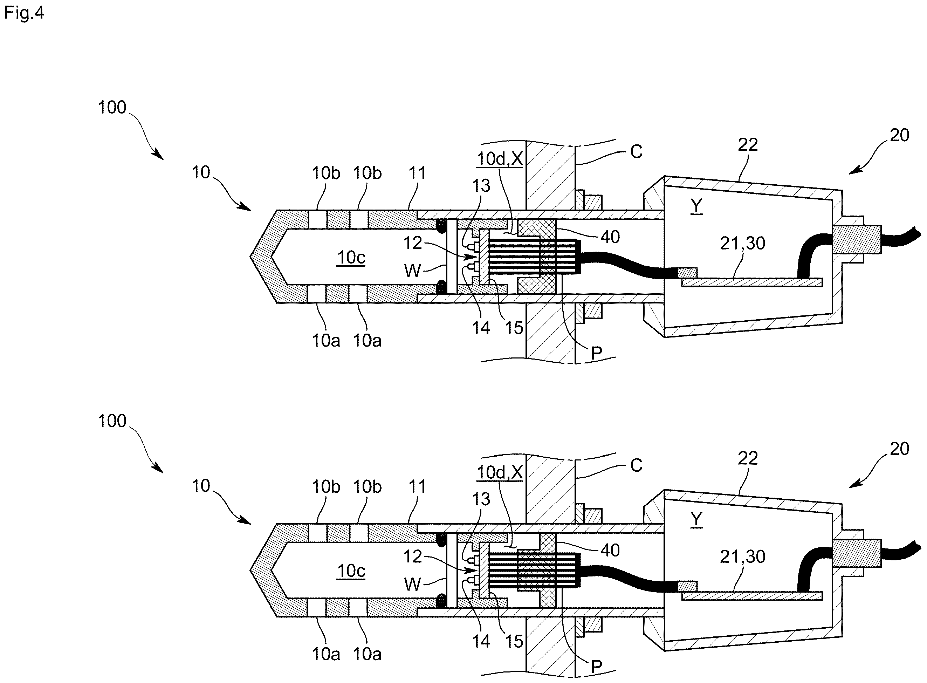

For example, in the above-mentioned embodiment, the bulkhead member 40 is cylindrical, however, it may be concave or convex as shown in Fig. 4, and in short, the bulkhead member 40 may be changed to various shapes as far as the entire circumference of the outer peripheral surface of the bulkhead member 40 is airtightly attached to the inner peripheral surface of the casing 11.

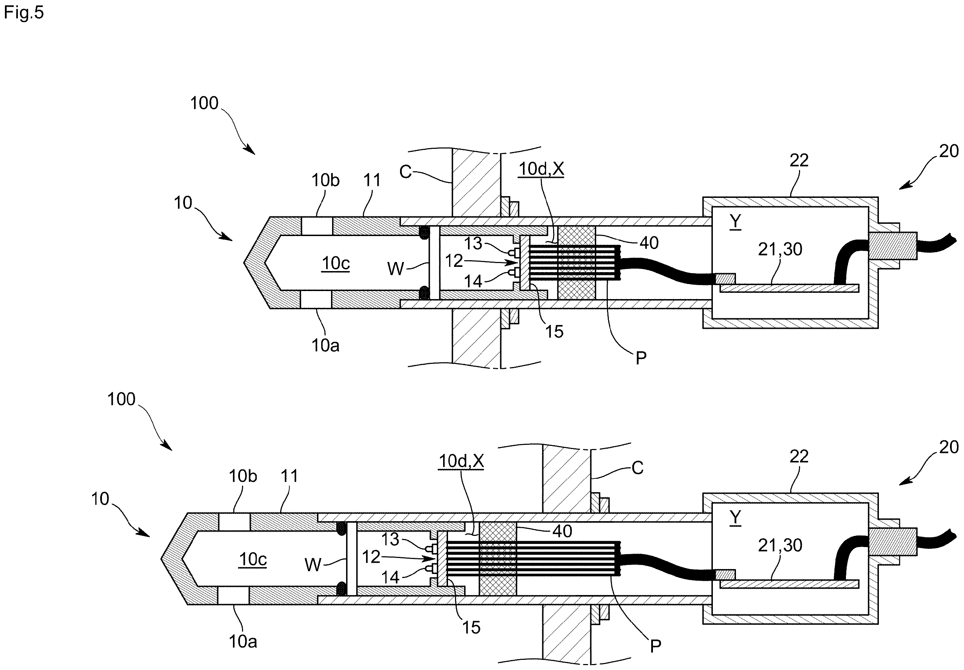

In addition, the explosion-proof area (X) may extend partly to the outside of the crankcase (C) as shown in an upper part of Fig. 5, and the non-explosion-proof area (Y) may extend partly to the inside part of the crankcase (C) as shown in a lower part of Fig. 5.

It is a matter of course that the present claimed invention is not limited to the above-mentioned embodiments, and various other variations are possible without departing from the spirit of the invention.

### Possible applications in industry

In accordance with the present claimed invention, it is possible to provide an unprecedented explosion-proof oil mist detection device without incurring higher costs.

## Claims

1. An oil mist detection device that detects oil mist in a casing and that comprises
a sensor unit that is housed in the casing and arranged in an explosion-proof area that requires explosion-proof measures,
a wiring member that is connected to the sensor unit,
a barrier circuit that is connected to the sensor unit through the wiring member, and
a bulkhead member through which the wiring member penetrates and that is housed in the casing and that seals the explosion-proof area, wherein
the barrier circuit is arranged in a non-explosion-proof area that is isolated from the explosion-proof area by the bulkhead member.

2. The oil mist detection device described in claim 1, wherein a plurality of the wiring members connect the sensor unit and the barrier circuit in a state wherein each of the wiring members is non-contact.

3. The oil mist detection device described in claim 1 or 2, wherein the wiring member is a single wire.

4. The oil mist detection device described in either one of claim 1 through claim 3, wherein the wiring member connects the sensor unit and the barrier circuit without being covered by an insulating member.

5. The oil mist detection device described in either one of claim 1 through claim 4, wherein the wiring member airtightly penetrates the bulkhead member.

6. The oil mist detection device described in either one of claim 1 through claim 5, wherein the bulkhead member is an elastic body made of resin.

7. The oil mist detection device described in either one of claim 1 through claim 6, wherein the barrier circuit limits energy supplied to the sensor unit.
